(19) Europäisches Patentamt
European Patent Office
Office européen des brevets

(11) Publication number: **0 002 312**
**B1**

(12) **EUROPEAN PATENT SPECIFICATION**

(45) Date of publication of patent specification: **03.02.82**

(21) Application number: **78300519.2**

(22) Date of filing: **19.10.78**

(51) Int. Cl.³: **C 07 D 498/04,**
A 61 K 31/42, A 61 K 31/43
//(C07D498/04, 263/00,
205/00), (A61K31/43, 31/42),
(A61K31/42, 31/545)

(54) **Derivatives of clavulanic acid and pharmaceutical compositions containing them.**

(30) Priority: **26.11.77 GB 4931577**
**26.11.77 GB 4931677**

(43) Date of publication of application:
**13.06.79 Bulletin 79/12**

(45) Publication of the grant of the European patent:
**03.02.82 Bulletin 82/5**

(84) Designated Contracting States:
**BE CH DE FR GB NL SE**

(56) References cited:
**FR - A - 2 359 144**

(73) Proprietor: **BEECHAM GROUP LIMITED**
**Beecham House Great West Road**
**Brentford, Middlesex (GB)**

(72) Inventor: **Liverman, Michael**
**21 Oakwood Close**
**Redhill Surrey (GB)**
Inventor: **Veal, Kenneth Thomas**
**Oakfield Lindens Close Effingham**
**Leatherhead Surrey (GB)**
Inventor: **Richardson, Kenneth**
**26 Hillcrest Drive Ashington**
**Pulborough Sussex (GB)**
Inventor: **Tedder, John Martin**
**40 Horley Road**
**Redhill Surrey (GB)**

(74) Representative: **Cole, William Gwyn, Dr. et al,**
**Beecham Pharmaceuticals Research Centre Yew**
**Tree Bottom Road**
**Epsom, Surrey KT18 5XQ (GB)**

EP 0 002 312 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Derivatives of clavulanic acid and pharmaceutical compositions containing them

The present invention relates to certain salts of some ethers of clavulanic acid, to a process for their preparation, to compositions containing them and to their use as intermediates.

Belgian Patent No. 847045 discloses inter alia that salts of ethers of clavulanic acid including amine salts such as monoalkylamine salts may be used to enhance the effectiveness of penicillins and cephalosporins in the treatment of bacterial infections such as those caused by $\beta$-lactamase producing strains of gram-negative and gram-positive bacteria such as *Klebsiella aerogenes* and *Staphylococcus aureus*. However the previously described salts, such as the sodium or potassium salts of the said ethers, tend to have relatively poor storage properties. It has now been found that certain new salts of these ethers have improved storage properties. In addition these new salts are useful in the preparation of the known alkali and alkaline earth metal salts of the ethers.

Accordingly the present invention provides the compounds of the formula (I):

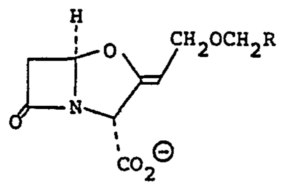

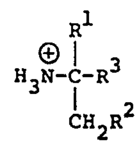

(I)

wherein

R is a hydrogen atom or a methyl, methoxyl, hydroxymethyl, or nitrile group; $R^1$ is a hydrogen atom or a lower alkyl, aralkyl, phenyl or inertly substituted lower alkyl, aralkyl or phenyl group; $R^2$ is a hydrogen atom or a lower alkyl, aralkyl, phenyl or inertly substituted lower alkyl, aralkyl or phenyl group; and $R^3$ is lower alkyl, aralkyl, phenyl or inertly substituted lower alkyl, aralkyl or phenyl group; any of said groups $R^1$, $R^2$ and $R^3$ being optionally interlinked to form a ring of 5—7 ring atoms.

When used herein the term 'lower' means that the group contains up to 6 carbon atoms. When used herein the term 'aralkyl' means a lower alkyl group substituted by a phenyl or inertly substituted phenyl group.

Suitable inert substituents include halogen, lower alkoxyl, lower acyloxyl, lower esterified carboxyl and the like groups. Suitably 1, 2 or 3 such substituent groups are present, more suitably 1 or 2 and preferably not more than one such substituent group is present.

Normally and preferably the amine of the formula (II):

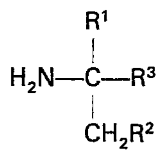

(II)

from which the salt of the formula (I) is notionally derivable is a non-toxic amine.

Most suitably $R^1$ is a lower alkyl group; most suitably $R^2$ is a hydrogen atom or a lower alkyl group; most suitably $R^3$ is a lower alkyl or aralkyl group; any of said groups being linked to form a 5—7 membered ring.

Most suitably the compound of the formula (II) does not contain a chiral centre.

Suitably R is methoxyl. Suitably R is nitrile. Suitably R is hydroxymethyl. More suitably R is methyl. Most suitably R is hydrogen.

Suitably the salt of the formula (I) is derivable from an amine of the formula (II) which contains 4—16 carbon atoms, more suitably 4—12 carbon atoms and preferably 4—10 carbon atoms.

Suitably the salt of the formula (I) is derivable from 2-amino-2-methylpropane, 2-amino-2-methylbutane, 2-amino-2-methylpentane, 2-amino-2-methylhexane, 2-amino-2-methylheptane, 2-amino-2-methyloctane, 3-amino-3-methylpentane, 1-amino-1-methylcyclohexane, 1-amino-spiro-adamantane and other similar amines.

Most suitably the amine of the formula (II) is tertiarybutylamine (that is $R^1$ and $R^3$ are methyl groups and $R^2$ is a hydrogen atom).

From the foregoing it will be realised that one particularly suitable compound of this invention is the tertiarybutylamine salt of the methyl ether of clavulanic acid. It will also be realised that another particularly suitable compound of this invention is the tertiarybutylamine salt of the ethyl ether of clavulanic acid.

The preceding compounds are particularly suitable when crystalline.

The present invention also provides pharmaceutical compositions which comprise a compound of the formula (I) as hereinbefore defined and a pharmaceutically acceptable carrier.

The compositions of this invention may be presented in orally or parenterally or other convenient

form. Generally the composition will be adapted for oral administration to a human although injectable forms and veterinary compositions may also be prepared.

Normally the composition of this invention will be in unit dosage form containing from about 20 mg to 250 mg, for example about 50, 75, 100, 125, 150 or 200 mg of a compound of the formula (I). Such compositions will usually be administered from 2 to 6 times daily, for example 3 or 4 times daily so that the daily dose is from about 60 mg to 1000 mg, more usually from about 200 mg to about 750 mg.

Desirably the composition of this invention will also comprise a penicillin or cephalosporin. Most suitably the ratio of compound of this invention to penicillin or cephalosporin is from 1:10 to 10:1, more usually 2:1 to 1:5, for example about 1:1, 1:2, 1:3 or 1:4 (by weight).

Suitable penicillins and cephalosporins for inclusion in such compositions include those named in Belgian Specifications Nos. 827926 or 847045.

Particularly suitable penicillins for inclusion in the compositions of this invention include ampicillin, amoxycillin and their pro-drugs such as their in-vivo hydrolysable esters. Preferred forms of these penicillins for inclusion in the compositions of this invention include ampicillin anhydrate, ampicillin trihydrate and amoxycillin trihydrate of which amoxycillin trihydrate is preferred. Other favoured forms of the penicillins include the pharmaceutically acceptable salts of ampicillin and amoxycillin such as their sodium salts. Yet other favoured forms of the penicillins include hydrochloride or other pharmaceutically acceptable salts of the pivaloyloxymethyl, phthalidyl or $\alpha$-ethoxycarbonyloxyethyl esters of ampicillin.

Other particularly suitable penicillins for inclusion in the compositions of this invention include carbenicillin, ticarcillin and their pro-drugs such as their in-vivo hydrolysable esters. Suitable forms of these penicillins include the di-sodium salts of carbenicillin or ticarcillin and the sodium salts of the phenyl, lower alkylphenyl or indanyl esters of carbenicillin or ticarcillin.

Penicillins suitable for inclusion in orally administrable compositions of this invention include benzylpenicillin, phenoxymethylpenicillin, propicillin, amoxycillin, ampicillin, epicillin, azidocillin, cyclacillin, and other orally active penicillins and their salts and *in-vivo* hydrolysable esters and aldehyde and ketone adducts of those penicillins containing a 6-$\alpha$-aminoacylamido side chain and their salts. Suitable penicillin *in-vivo* hydrolysable esters includer the acetoxymethyl, pivaloyloxymethyl, $\alpha$-ethoxycarbonyloxyethyl and phthalidyl esters of ampicillin or amoxycillin or the phenyl tolyl and indanyl $\alpha$-esters of carbenicillin and ticarcillin and salts thereof. Suitable aldehyde and ketone adducts of penicillins containing a 6-$\alpha$-aminoacylamido side chain include the formaldehyde and acetone adducts of metampicillin and hetacillin and their salts. Suitable penicillins for inclusion in injectably or infusably administrable compositions include the acceptable salts of benzylpenicillin, phenoxymethylpenicillin, carbenicillin, propicillin, azidocillin, ampicillin, amoxycillin, epicillin, azlocillin, mezlocillin, ticarcillin, cyclacillin and other known penicillins.

Cephalosporins suitable for inclusion in orally administrable compositions of this invention include cephalexin, cephradine, cephaloglycine, cephaparole and their salts and other known cephalosporins and their salts and *in-vivo* hydrolysable esters and aldehyde and ketone adducts of those cephalosporins containing a 7-$\alpha$-aminoacylamido side chain and their salts. Suitable cephalosporins for inclusion in the injectable or infusible compositions of this invantion include the salts of cephaloridine, cephalothin, cefazolin, cephalexin, cephacetril, cephamandole, cephaprin, cefuroxime, cephradine, cephaloglycine, cephatrizine, and other known cephalosporins.

Particularly suitable forms of the compositions of this invention are those which do not contain high levels of residual water. Most suitably such compositions are prepared from dry materials so that the final compositions contain less than 5% moisture, more suitably less than 3% moisture and preferably less than 2% moisture, for example 1% or less of moisture.

Most suitably the compositions of this invention are packaged in moisture-excluding forms such as closed containers, foil lined packs and the like. Such packs may also contain a desiccant such as silica gel or a molecular sieve.

The present invention also provides a process for the preparation of the compounds of the formula (I) which process comprises the reaction of a compound of the formula (III):

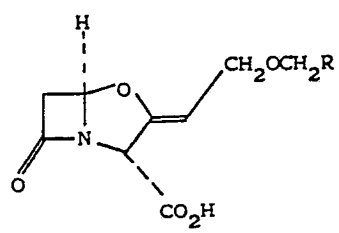

(III)

wherein

R is as defined in relation to formula (I), with a compound of the formula (II):

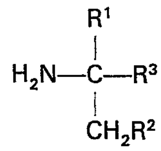

(II)

wherein

$R^1$, $R^2$ and $R^3$ are as defined in relation to formula (I).

The neutralisation of the acid (III) with the amine (II) may be brought about under conventional conditions for such reactions, for example the reaction is performed at a non-extreme temperature (usually from about 0° to 30°C, e.g. 15—25°C) and in an inert organic solvent such as an ether, halohydrocarbon, ketone or ester solvent, for example tetrahydrofuran, diethylether, methylene dichloride, chloroform, acetone, ethyl acetate or mixtures of such solvents.

The desired salt can generally be recovered from the reaction mixture by filtration (after concentration if necessary). The salt may then be dried for example in vacuo. If desired the salt may be recrystallised, for example from a lower alkanol such as ethanol. One crystallisation is generally sufficient to yield a salt of at least 95% purity (weight/weight).

The present invention also provides a process for the preparation of a salt of the formula (I) which process comprises the displacement of an alternative cation from an alternative salt of a compound of the formula (III) by a protonated amine of the formula (II). Normally this will involve the use of a salt of the amine with a cation exchange resin.

Suitable cation exchange resins for use in this process are cross-linked polystyrene-divinylbenzene co-polymers substituted by acid groups in the form of a salt of the desired amine of the formula (II). The preferred acid group is the sulphonic acid group. The resins chosen will generally have 2—20% cross-linking and usually 4—10% cross-linking for example about 8% cross-linking. The resin is usually in the form of beads, for example spherical beads of 14—52 U.K. mesh size.

Suitable resins include the desired amine salt (e.g. t-butylamine) form of Amberlite resins such as IR—120, IR—121, IR—122, 200, 200C, 252; Dowex resins such as 50WX1, 50WX2, 50X4, 50WX8, 5X10, WX10, 50WX16; Bio Rad resins such as AG 50WX1, 40WX2, AG 50WX4, AG 50WX8, AG 50WX12; Ionac resins such as C250, C258 or C255 and Zerolit resins such as 225, 325, 425, 525 or 625 (Amberlite, Dowex and Bio Rad are registered Trade Marks).

The resin is normally used in large excess. The quantity of resin used should be sufficient to provide an exchange capacity at least 10 times, more suitably at least 20 times and preferably at least 30 times of the total cations applied to the solution of the salt of clavulanic acid ether.

In use the resin is in a bed through which the solution is percolated. Generally this bed is in the form of a column.

The concentration of the solution of the salt to be exchanged is not critical but very dilute solutions (for example, less than 1% w/v) should be avoided as low concentrations can lead to inconveniently low loading and incomplete exchange. Similarly very high concentrations (for example those approaching saturation) should be avoided as such solutions can have too high a viscosity for easy use. In general for most salts concentration 2—30% w/v are acceptable, a range of about 5 to 25% being more suitable and a range of about 8 to 20% being preferred, for example about 10%.

The solution to be exchanged is normally applied slowly to the top of the column. The band is then allowed to percolate slowly into the top of the resin after which a little water is applied to wash the band a short way into the resin. The remaining necessary water is then slowly run through the column so that the band passes through the column in as tight a band as conveniently possible.

The presence of the desired salt in the eluate is usually readily detectable by a change in the refractive index of the eluate. This may be determined using a refractometer or visually, for example by the presence of striations. Alternatively fractions may be taken and tested in convenient manner, for example by spotting on a thin layer chromatography plate and spraying with permanganate which is decoloured by the β-lactam containing compound or by methods using the enzyme inhibitory effects of the material.

The desired salt of the amine of formula (I) may be obtained from solution in conventional manner, for example from a relatively concentrated solution by the slow addition of a water miscible organic solvent such as acetone until crystallisation commences or by concentrating under reduced pressure to a syrup followed by adding small quantities of acetone, acetonitrile, acetone/ether or the like to initiate crystallisation.

The present invention provides a process for the preparation of the sodium, potassium, calcium or magnesium salt of a compound of the formula (III) which process comprises the reaction of a compound of the formula (I) with a sodium, potassium, calcium or magnesium salt of a cation exchange resin.

Suitable cation exchange resins for use in this process are cross-linked polystyrene-divinylbenzene co-polymers substituted by acid groups in the form of the desired alkali metal or alkaline earth metal salt. The preferred acid group is the sulphonic acid group. The resins chosen will generally have 2—20% cross-linking and usually 4—10% cross-linking for example about 8% cross-linking. The

resin is usually in the form of beads, for example spherical beads of 14—52 U.K. size.

Suitable resins include the desired alkali metal or alkaline earth metal form of Amberlite resins such as IR—120, IR—121, IR—122, 200, 200C, 252; Dowex resins such as 50WX1, 50WX2, 50X4, 50WX8, 5X10, WX10, 50WX16; Bio Rad resins such as AG 50WX1, 40WX2, AG 50WX4, AG 50WX8, AG 50WX12; Ionac resin such as C250, C258 or C255 and Zerolit resins such as 225, 325, 425, 525 or 625.

The resin is normally used in large excess. The quantity of resin used should be sufficient to provide an exchange capacity at least 10 times, more suitably at least 20 times and preferably at least 30 times of the total cations applied to the solution of the salt of clavulanic acid ether.

In use the resin is in a bed through which the solution is percolated. Generally this bed is in the form of a column.

The concentration of the solution of the salt to be exchanged is not critical but very dilute solutions (for example, less than 1% w/v) should be avoided as low concentrations can lead to inconveniently low loading and incomplete exchange. Similarly very high concentrations (for example those approaching saturation) should be avoided as such solutions can have too high a viscosity for easy use. In general for most salts concentration 2—30% w/v are acceptable, a range of about 5 to 25% being more suitable and a range of about 8 to 20% being preferred, for example about 10%.

The solution to be exchanged is normally applied slowly to the top of the column. The band is then allowed to percolate slowly into the top of the resin after which a little water is applied to wash the band a short way into the resin. The remaining necessary water is then slowly run through the column so that the band passes through the column in as tight a band as conveniently possible.

The presence of the desired salt in the eluate is usually readily detectable by a change in the refractive index of the eluate. This may be determined using a refractometer or visually, for example by the presence of striations. Alternatively fractions may be taken and tested in convenient manner, for example by spotting on a thin layer chromatography plate and spraying with permanganate which is decoloured by the $\beta$-lactam containing compound or by methods using the enzyme inhibitory effects of the material.

The desired alkali metal or alkaline earth metal salt may be obtained from solution in conventional manner, for example from a relatively concentrated solution by the slow addition of a water miscible organic solvent such as acetone until crystallisation commences or by concentrating under reduced pressure to a syrup followed by adding small quantities of acetone, acetonitrile, acetone/ether or the like to initiate crystallisation.

Most suitably crystallisation is initiated at an ambient or slightly elevated temperature, for example, 12—30°C, more suitably 20—25°C. Once crystallisation has begun the mixture may be cooled, for example to about −5°C to −10°C until no further crystals appear.

Once the desired crystals have formed they may be filtered off and dried. Vigorous drying conditions (such as vacuum drying) are best avoided as they tend to lead to breakdown of the crystal structure and partially dehydrated crystals. Wet air should not be used for drying as it can lead to wet crystals. Drying should be effected at atmospheric pressure.

Suitably the preceding process is adapted to the preparation of a sodium salt.

Suitably the preceding process is adapted to the preparation of a potassium salt.

Suitably the preceding process is adapted to the preparation of a calcium salt.

Suitably the preceding process is adapted to the preparation of a magnesium salt.

Example 1

Preparation of t-butylamine salt of O-methyl clavulanic acid

p-Methoxybenzyl O-methyl clavulanate (20 g) in tetrahydrofuran (200 ml) was hydrogenated at $1.36 \times 10^5$Pa (5 p.s.i.) in the presence of 10% palladium on charcoal. The catalyst was filtered off and the filtrate evaporated until solid just appeared. Fresh tetrahydrofuran was added dropwise until the solid just redissolved and the solution diluted with ether (500 ml). The solution was stirred and t-butylamine (4.4 g) was added dropwise. After stirring for a further 2 hours at ambient temperature, the solid was filtered off, washed with ether and dried in vacuo to yield crude t-butylamino O-methyl clavulanate (15.2 g). This material was recrystallised from ethanol (150 ml) and ether (750 ml) to yield pure crystalline t-butylamine O-methyl clavulanate (11.9 g). (*Purity* 97.5% by imidazole assay, 95.7% by h.p.l.c. assay. Moisture 0.4%).

I.r. (major peaks) = 2650, 2530, 1784, 1692, 1640, 1570, 1400, 1391, 1296, 1279, 1188, 1147, 1075, 1015, 902, 736 cm.

20 CuKx
(x-ray major peaks) = 10.9 12.2 13.2 15.2 16.7 17.6 18.3 19.2 19.8 20.7 22.1 23.0 24.5 25.2 26.6

Example 2

Preparation of the t-butylamine salt of O-methyl clavulanic acid

The benzyl ester of O-methylclavulanic acid (39.2 g, 0.129 mole) in THF (400 ml) was

hydrogenated at 1.36 × $10^5$Pa (5 p.s.i.) in the presence of 10% Pd/C (13 g) until completion (ca 40 mins). The mixture was filtered through Celite and the resultant solution evaporated to a solid. (Celite is a registered Trade Mark). This was dissolved in the minimum quantity of THF and diluted with ether (1 l). To this stirred solution was added t-butylamine (9.45 g, 0.129 mole) and crystallization occurred in a short time. After one hour the solid was filtered off, washed with ether and dried in vacuo. Recrystallization from ethanol/ether afforded 27.1 g (73%) of white material of >95% purity.

Example 3

Preparation of the t-butylamine salt of O-ethylclavulanic acid

The p-methoxybenzyl ester of O-ethylclavulanic acid (47 g, 0.1355 mole) in THF (470 ml) was hydrogenated at 1.36 × $10^5$Pa (5 p.s.i.) in the presence of 10% Pd/C (15.1 g) until completion (ca 40 mins). The mixture was filtered through Celite and the resultant solution evaporated to an oil. This was dissolved in ether (800 ml) and to it was added t-butylamine (9.91 g, 0.1355 mole). Crystallization occured after a short time and after two hours at 4°C the solid was filtered off, washed with ether and dried in vacuo. Recrystallization from isopropanol/ether afforded 24 g (59%) of white material of >95% purity.

Ir. (major peaks) = 2660, 2530, 1809, 1690, 1626, 1586, 1399, 1305, 1296, 1195, 1093, 1048, 1025, 1000, 898, 743 cm.

Example 4

Preparation of the t-butylamine salt of O-methoxymethylclavulanic acid

The benzyl ester of O-methoxymethylclavulanic acid (83.3 g, 0.25 mole) was dissolved in THF (1250 ml) and water (20 ml) was added. 10% Pd/C (20 g) was used to catalyse the hydrogenolysis which was carried out under a pressure of 4.07 × $10^5$Pa (45 p.s.i.) for 1 hour. The reaction mixture was filtered and t-butylamine (27.2 ml = 18.25 g, 0.25 mole) was added. The solvent was evaporated in vacuo and final traces of water were removed under high vacuum. A thick yellow oil resulted, this was dissolved in the least volume of isopropyl alcohol and was shaken vigorously with anhydrous ether (3000 ml). A precipitate formed; after cooling at 5°C for 1 hour this was filtered, washed with ether and dried in vacuo to afford 48 g (62%) of an off-white solid whose nmr spectrum was consistent with required structure. (Purity 79%).

Ir. (major peaks) = 2660, 2530, 1805, 1698, 1628, 1588, 1397, 1306, 1295, 1197, 1101, 1038, 895, 745 cm.

Example 5

Composition

a) The active ingredient, lubricant and filler may be blended together, granulated and tabletted on a conventional rotary machine to yield 5000 tablets each containing approximately:

| | |
|---|---|
| t-butylamine O-methylclavulanate | 125 mg |
| Magnesium stearate | 1 mg |
| Microcrystalline cellulose | 125 mg |

b) A composition analogous to that described in a) may be prepared by replacing the t-butylamine O-methylclavulanate with t-butylamine O-ethylclavulanate.

c) The active ingredients, lubricants and filler may be blended together, granulated and tabletted on a conventional rotary machine to yield 5000 tablets each containing approximately:

| | |
|---|---|
| Amoxycillin trihydrate* | 300 mg |
| t-butylamine O-methylclavulanate | 125 mg |
| magnesium stearate | 3 mg |
| microcrystalline cellulose | 125 mg |

(*containing equivalent of 250 mg pure free amoxycillin)

(These formulations may be prepared under a dry atmosphere if desired, for example at less than 30% relative humidity).

Example 6

Preparation of the Sodium salt of O-methyl clavulanic acid

O-methyl clavulanic acid t-butylamine salt (10.9 g) in water (5 ml/gm) was passed down an ionexchange column Amberlite IR 120 $Na^{\oplus}$ form (100 ml wet resin). Fractions containing the sodium salt were combined and evaporated to dryness at room temperature. The residue was dissolved in acetone (100 ml) and added with vigorous stirring to ether (1 litre). After stirring 1 hour at ambient temperature the precipitated sodium salt was filtered off, washed with ether and dried in vacuo to yield sodium O-methyl clavulanate (8.2 g) (Purity 87.4% by imidazole assay; moisture 6.2%).

**Claims**

1. A compound of the formula (I):

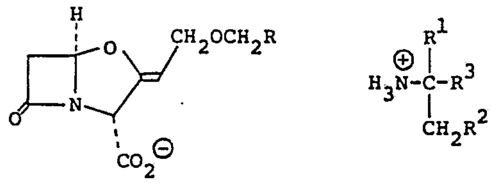

wherein

R is a hydrogen atom or a methyl, methoxyl, hydroxymethyl, or nitrile group; $R^1$ and $R^2$ are independently a hydrogen atom or a $C_{1-6}$ alkyl, phenyl ($C_{1-6}$) alkyl, phenyl or inertly substituted $C_{1-6}$ alkyl, phenyl ($C_{1-6}$) alkyl or phenyl group; and $R^3$ is $C_{1-6}$ alkyl, phenyl ($C_{1-6}$) alkyl, phenyl or inertly substituted $C_{1-6}$ alkyl, phenyl ($C_{1-6}$) alkyl or phenyl group; any of said groups $R^1$, $R^2$, and $R^3$ being optionally interlinked to form a ring of 5—7 ring atoms.

2. A compound as claimed in claim 1 wherein the salt is derivable from 2-amino-2-methylbutane, 2-amino-2-methylpentane, 2-amino-2-methylhexane, 2-amino-2-methylheptane, 2-amino-2-methyloctane, 3-amino-3-methylpentane, 1-amino-1-methylcyclohexane or 1-amino-spiro-adamantane.

3. A compound as claimed in claim 1 wherein $R^1$ is a methyl group, $R^2$ is a hydrogen atom and $R^3$ is a methyl group.

4. The tertiarybutylamine salt of clavulanic acid methyl ether.

5. The tertiarybutylamine salt of clavulanic acid ethyl ether.

6. A compound as claimed in any of claims 1—5 in crystalline form.

7. A pharmaceutical composition which comprises a compound as claimed in any of claims 1—6 and a pharmaceutically acceptable carrier.

8. A composition as claimed in claim 7 adapted for oral administration to a human.

9. A composition as claimed in claims 7 or 8 in the unit-dose form containing 20 mg to 250 mg of the compound as claimed in any of claims 1—6.

10. A composition as claimed in any of claims 7—9 which also comprises a penicillin or cephalosporin.

11. A composition as claimed in claim 10 which comprises amoxycillin.

12. A composition as claimed in claim 11 wherein the amoxycillin is present as amoxycillin trihydrate.

**Patentansprüche**

1. Eine Verbindung der Formel (I)

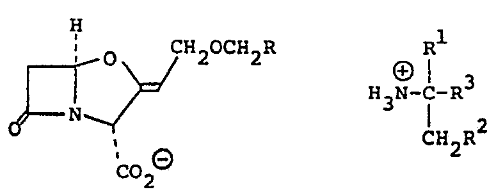

(I)

in der R ein Wasserstoffatom oder eine Methyl-, Methoxy-, Hydroxymethyl- oder Nitrilgruppe ist, $R^1$ und $R^2$ unabhängig voneinander ein Wasserstoffatom oder einen $C_{1-6}$-Alkyl-, Phenyl-($C_{1-6}$)-alkyl-, Phenylrest oder einen mit einem inerten Rest substituierten $C_{1-6}$-Alkyl-, Phenyl-($C_{1-6}$)-alkyl- oder Phenylrest bedeuten und $R^3$ ein $C_{1-6}$-Alkyl-, Phenyl-($C_{1-6}$)-alkyl-, Phenylrest oder ein mit einem inerten Rest substituierter $C_{1-6}$-Alkyl-, Phenyl-($C_{1-6}$)-alkyl- oder Phenylrest ist, wobei die Reste $R^1$, $R^2$ und $R^3$ gegebenenfalls zu einem Ring mit 5 bis 7 Ringatomen verbunden sein können.

2. Eine Verbindung gemäß Anspruch 1, wobei sich das Salz von 2-Amino-2-methylbutan, 2-Amino-2-methylpentan, 2-Amino-2-methylhexan, 2-Amino-2-methylheptan, 2-Amino-2-methyloctan, 3-Amino-3-methylpentan, 1-Amino-1-methylcyclohexan oder 1-Amino-spiro-adamantan ableitet.

3. Eine Verbindung gemäß Anspruch 1, wobei $R^1$ eine Methylgruppe, $R^2$ ein Wasserstoffatom und $R^3$ eine Methylgruppe ist.

4. Das tert.-Butylaminsalz des Clavulansäure-methyläthers.

5. Das tert.-Butylaminsalz des Clavulansäure-äthyläthers.

6. Eine Verbindung gemäß einem der Ansprüche 1—5 in kristalliner Form.

7. Eine pharmazeutische Zusammensetzung, die eine Verbindung gemäß einem der Ansprüche 1 bis 6 und einen pharmakologisch verträglichen Träger enthält.

8. Eine Zusammensetzung gemäß Anspruch 7, angepaßt für die orale Verabreichung an Menschen.

9. Eine Zusammensetzung gemäß den Ansprüchen 7 oder 8 in Form einer Einzeldosis, enthaltend 20 mg bis 250 mg der Verbindung gemäß einem der Ansprüche 1 bis 6.

10. Eine Zusammensetzung gemäß einem der Ansprüche 7 bis 9, die auch Penicillin oder Cephalosporin enthält.

11. Eine Zusammensetzung gemäß Anspruch 10, die Amoxycillin enthält.

12. Eine Zusammensetzung gemäß Anspruch 11, wobei das Amoxycillin als Amoxycillin-trihydrat vorliegt.

**Revendications**

1. Composé de formule (I)

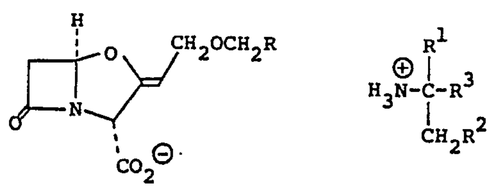

dans laquelle R est un atome d'hydrogène ou un groupe méthyle, méthoxy, hydroxyméthyle ou nitrile; $R^1$ et $R^2$ sont, indépendamment, un atome d'hydrogène ou un groupe alcoyle en $C_{1-6}$, phényl-alcoyle ($C_{1-6}$), phényle, ou bien alcoyle en $C_{1-6}$, phényl-alcoyle ($C_{1-6}$) ou phényle à substitution inerte, et $R^3$ est un groupe alcoyle en $C_{1-6}$, phényl-alcoyle ($C_{1-6}$), phényle, ou bien alcoyle en $C_{1-6}$, phényl-alcoyle ($C_{1-6}$) ou phényle à substitution inerte; un ou plusieurs de ces groupes $R^1$, $R^2$ et $R^3$ étant éventuellement reliés ensemble pour former un cycle ayant de 5 à 7 atomes cycliques.

2. Composé suivant la revendication 1, caractérisé en ce que le sel peut être dérivé du 2-amino-2-méthylbutane, du 2-amino-2-méthylpentane, du 2-amino-2-méthylhexane, du 2-amino-2-méthylheptane, du 2-amino-2-méthyloctane, du 3-amino-3-méthylpentane, du 1-amino-1-méthylcyclohexane ou du 1-amino-spiro-adamantane.

3. Composé suivant la revendication 1, caractérisé en ce que $R^1$ est un groupe méthyle, $R^2$ est un atome d'hydrogène et $R^3$ est un groupe méthyle.

4. Sel de butylamine tertiaire d'éther méthylique de l'acide clavulanique.

5. Sel de butylamine tertiaire de l'éther éthylique d'acide clavulanique.

6. Composé suivant l'une quelconque des revendication 1 à 5, sous forme cristalline.

7. Composition pharmaceutique, caractérisée en ce qu'elle renferme un composé suivant l'une quelconque des revendications 1 à 6 et un véhicule ou excipient pharmaceutiquement acceptable.

8. Composition suivant la revendication 7, caractérisée en ce qu'elle est adaptée en vue d'une administration orale aux êtres humains.

9. Composition suivant la revendication 7 ou 8 sous forme de dose unitaire contenant de 20 mg à 250 mg du composé suivant l'une quelconque des revendications 1 à 6.

10. Composition suivant l'une quelconque des revendications 7 à 9, caractérisée en ce qu'elle renferme également une pénicilline ou une céphalosporine.

11. Composition suivant la revendication 10, caractérisée en ce qu'elle renferme de l'amoxycilline.

12. Composition suivant la revendication 11, caractérisée en ce que l'amoxycilline est présente sous forme d'amoxycilline trihydrate.